# EUROPÄISCHE PATENTSCHRIFT

(11) **EP 1 341 490 B1**
(45) Veröffentlichungstag und Bekanntmachung des Hinweises auf die Patenterteilung: **23.02.2005**
(21) Anmeldenummer: 01995666.3
(22) Anmeldetag: 27.11.2001
(51) Int. Cl.: A61F 2/44

(54) **BANDSCHEIBENTEILERSATZIMPLANTAT**
PARTIAL INTERVERTEBRAL DISK REPLACEMENT IMPLANT
IMPLANT DE REMPLACEMENT DE DISQUE INTERVERTEBRAL PARTIEL

(30) Priorität: 13.12.2000 DE 10061975
(43) Veröffentlichungstag der Anmeldung: 10.09.2003
(73) Patentinhaber: ESKA Implants GmbH & Co., 23556 Lübeck (DE)
(72) Erfinder: GRUNDEI, Hans, 23556 Lübeck (DE); ARNOLD, Wolf, 98529 Suhl (DE)
(74) Vertreter: Fuchs Mehler Weiss & Fritzsche
(86) Internationale Anmeldenummer: PCT/EP2001/013782
(87) Internationale Veröffentlichungsnummer: WO 2002/047586

(56) Entgegenhaltungen:
- EP-A- 0 282 161
- WO-A-00/13620
- WO-A-99/32054
- FR-A- 2 794 362

## Beschreibung

Die vorliegende Erfindung betrifft ein Bandscheibenteilersatzimplantat zur Behandlung von Bandscheibenläsionen, bei denen ein Ersatz der vollständigen Bandscheibe durch ein Implantat, beispielsweise gemäß der DE-42 13 771 C1 (noch) nicht angezeigt ist. Vielmehr zielt das Teilersatzimplantat ab auf eine lokale Ausbesserung der lädierten Bandscheibe, um negative Konsequenzen aus der Läsion zu vermeiden.

Aus der DE-43 23 595 C1 ist ein solches Bandscheibenteilersatzimplantat bekannt, welches ausschließlich als Entlastungsteil wirkt. Es besteht aus einer Hülse aus elastischem Material sowie aus einem metallischen Körper mit einem Zapfen, welcher an einer Abschlußplatte angebracht ist. Die Hülse weist im Innenraum einen Aufnahmeraum für diesen Zapfen auf, so daß die Abschlußplatte bei der in die Hülse gesetzten Zapfen im wesentlichen an der Stirnseite der Hülse anliegt und im wesentlichen bündig mit dieser abschließt.

Der bekannte Bandscheibenteilersatz besteht also im wesentlichen aus einer metallarmierten elastischen Hülse. In der Regel werden pro Bandscheibe zwei derartige Implantate eingesetzt, wobei etwa in Richtung der Fortsätze der Wirbelkörper jeweils eine Bohrung in die beschädigte Bandscheibe eingebracht wird. Das Implantat ist so dimensioniert, daß es sich ausschließlich im Bereich der Bandscheibe erstreckt, nicht jedoch in den knöchernen Bereich der benachbarten Wirbelkörper. Eine dreidimensionale offenmaschige Raumnetzstruktur an der Peripherie der Abschlußplatte soll dafür sorgen, daß das Implantat in seiner Lage fixiert wird, indem Bindegewebe in sie hineinwächst.

Der aus der besagten Druckschrift bekannte Bandscheibenteilersatz leistet gute Dienste bei nur leicht lädierten Bandscheiben. Bei stärkeren Läsionen, die aber noch keine Implantation einer Bandscheibenvoilendoprothese rechtfertigen würde, reicht die Ablastung und die Ortsstabilität des Implantates jedoch nicht aus.

Ein gattungsgemäßes Bandscheibenteilersatzimplantat ist bekannt aus der WO-A-99/32054. Dieses Implantat besteht aus einer im wesentlichen zylindrischen, zweigeteilten Hülse und aus einer Einlage aus elastischem Material, welche die beiden Hülsenteile voneinander trennt und zwischen diesen einen elastischen Puffer bildet.

Die Einlage ist mit den Hülsenteilen über einen Silikonklebstoff o. dgl. verbunden. Ihre Stirnseiten sind abgeflacht. Es hat sich nun ergeben, daß dieses Implantat relativ umständlich zu handhaben ist, da es schwierig in die Bohrung in der beschädigten Bandscheibe zu platzieren ist.

Vor diesem Hintergrund ist es die Aufgabe der vorliegenden Erfindung, ein Bandscheibenteilersatzimplantat anzugeben, dessen Handhabung gegenüber dem gattungsgemäßen Implantat deutlich verbessert ist.

Gelöst wird diese Aufgabe durch das Teilersatzimplantat mit den Merkmalen des Anspruchs 1. Vorteilhafte Weiterbildungen ergeben sich aus den Unteransprüchen.

Demgemäß wird vorgeschlagen, daß die Teile der Hülse einseitig aneinander angelenkt sind und daß das distale Ende ballig ausgebildet ist.

Die beiden Hülsenteile stehen also bis auf den Bereich, in dem beide Teile aneinander angelenkt sind, nicht miteinander in Verbindung. Vielmehr bildet die elastische Einlage einen Puffer zwischen den beiden Hülsenteilen. Die Anlenkung der einen Hülse an die andere, vorzugsweise im distalen Bereich, dient dazu, das Teilersatzimplantat als Einheit handhaben zu können.

Im proximalen Bereich ist eine Verkoppelung beider Hülsenteile, die elastische Einlage überspannend, nicht vonnöten. In der Praxis nämlich wird die Bohrung in die beschädigte Bandscheibe stets einen etwas kleineren Durchmesser als das Implantat haben, so daß dieses nach dem Einsetzen in die Bohrung aufgrund der in der Wirbelsäule des Patienten herrschenden Kräfte ohnehin zusammengepreßt wird.

Das distale Ende des Implantates ist ballig ausgebildet, also abgerundet. Dies erleichtert den Einsatz des Implantates in die eingebrachte Bohrung in die Bandscheibe und die benachbarten Wirbelkörper.

Das Implantat hat insgesamt einen größeren Durchmesser als die Bandscheibe breit ist. Dies vor dem Hintergrund, daß mit Teilen der Außenwandung der beiden Hülsenteile ein knöcherner Kontakt entstehen soll, so daß die beiden Hülsenteile jeweils mit den cranialen und caudalen Kontaktbereichen der anlegenden Wirbelkörper in Kontakt kommen. Vorzugsweise weisen die beiden Hülsenteile daher in diesen Bereichen eine offenmaschige dreidimensionale Raumnetzstruktur auf, in welche und durch welche hindurch Knochenmaterial zur dauerhaften Fixierung der vorzugsweisen metallischen Hülsenteile ein- bzw. durchwachsen soll. Zwischen den dann mit den benachbarten Wirbelkörpern fest verwachsenen Hülsenteile befindet sich dann weiterhin die elastische Einlage, welche die Funktion der lädierten Bandscheibe an der Implantationsstelle übernimmt, d. h. unter anderem auch eine abpuffernde Funktion.

Gemäß einer vorteilhaften Weiterbildung weist die elastische Einlage einen Mittelwulst auf, der in entsprechend ausgebildete Mulden in den beiden Hülsenhälften formschlüssig eingreift. Einerseits erhöht der Mittelwulst lokal die Dicke des elastischen Materials und unterstützt somit die abpuffernde Funktion. Andererseits wird durch das formschlüssige Eingreifen in die Mulden in den Hülsenteilen dafür gesorgt, daß die elastische Einlage sicher zwischen den Hülsenteilen verharrt und nicht durch Mikrobewegungen im Laufe der Zeit aus der vorgesehenen Lage auswandert, so daß sich dann die beiden (metallischen) Hülsenteile an unkontrollierten Stellen berühren würden.

Proximal ist in einem Teil der Hülse eine Aufnahme für das Einsatzinstrument vorgesehen. Wenn der Kopf des Einsatzinstrumentes ein Sechskant ist, so ist die Aufnahme vorzugsweise als Innensechskant ausgebildet.

Die Erfindung wird anhand eines Ausführungsbeispiels näher erläutert. Hierbei zeigt:
Fig. 1 in Explosionsdarstellung das Oberteil (a), die Zwischenlage (b) und das Unterteil (c) des Implantates,
Fig. 2 die Aufsicht auf das zusammengesetzte Implantat von oben, und
Fig. 3 die Seitenansicht des zusammengefügten Implantates.

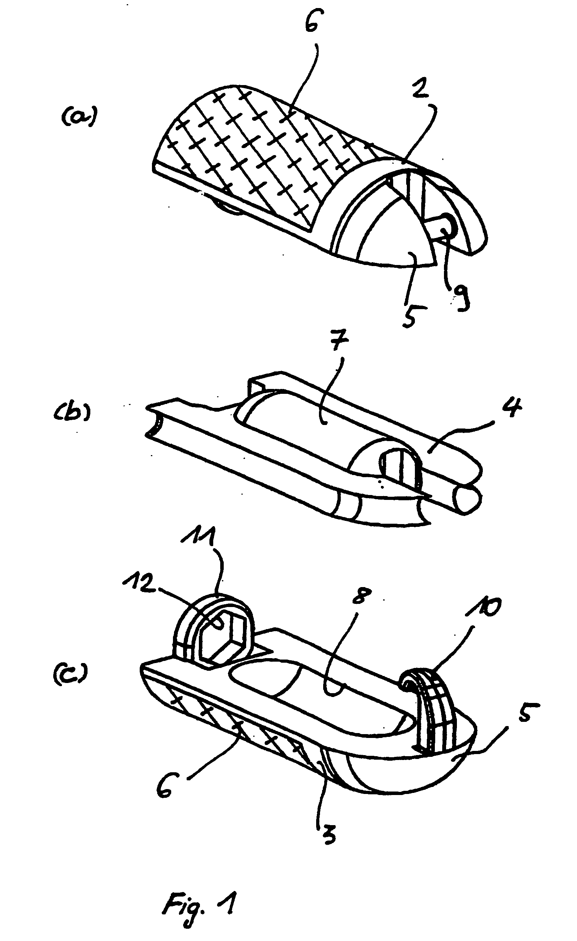

Einen ersten Überblick verschafft Fig. 1. Daraus ist ersichtlich, daß das erfindungsgemäße Implantat besteht aus zwei (metallischen) Hülsenteilen 2 und 3. Beide sind so ausgebildet, daß die Hülsen im zusammengesetzten Zustand ein distales, ballig ausgebildetes Ende aufweisen.

Das Unterteil 3 des Implantates weist distalseitig ein Koppelungselement 10 auf, welches mit einem Koppelungselement 9 in Form eines Quersteges im Oberteil 2 zusammenarbeitet, derart, daß beide Hülsenteile 2 und 3 aneinander angelenkt werden können.

Zwischen beide Hülsenteile wird die elastische Einlage 4 gesetzt, die vorliegend einen Mittelwulst 7 aufweist, welcher in entsprechenden Mulden 8 in den Hülsenteilen 2 und 3 gesetzt werden kann. Der Zusammenbau des Implantates geht nun in der Weise vonstatten, daß die elastische Einlage 4 zunächst auf das Unterteil 3 gesetzt wird, wobei der Mittelwulst 7 in die Mulde 8 im Unterteil 3 greift. Das Oberteil wird mit seinem Steg 9 gewissermaßen unter das Koppelungselement 10 des Unterteil in Form eines Haken gehakt und in Richtung auf das Hülsenteil 3 nach unten verschwenkt, so daß die Einlage 4 zwischen den Hülsenteilen 2 und 3 eingeklemmt wird.

Die Außenwandung der Hülsenteile 2 und 3 sind in dem Bereich, in dem sie in Kontakt mit dem benachbarten knöchernen Wirbelkörpern kommen, mit einer offenmaschigen, dreidimensionalen Raumnetzstruktur 6 versehen, durch welche hindurch Knochentrabekel zu dauerhaften Fixation im knöchernen Lager wächst.

Proximal ist eine Aufnahme 11 im unteren Hülsenteil 3 angeformt, in der vorliegend eine Innensechskant 12 ausgebildet ist, der mit einem Außensechskant eines Setzinstrumentes (nicht dargestellt), zusammenarbeitet.

Fig. 2 zeigt die Aufsicht auf das zusammengesetzte Implantat 1. Deutlich erkennbar ist das Koppelungselement 10, wie dieses den Bolzen 9 des oberen Gehäuseteils umfaßt.

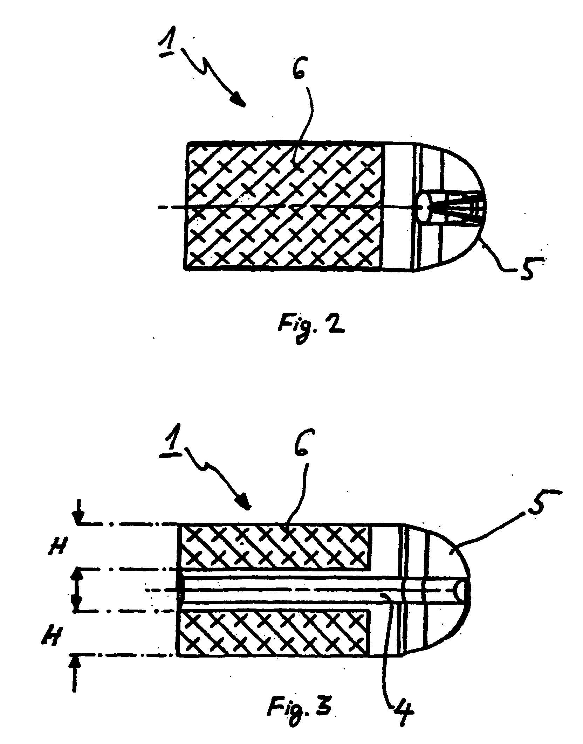

Fig. 3 zeigt die Seitenansicht des zusammengefügten Implantates. Deutlich wird daraus, daß das Implantat 1 nur dort die offenmaschige, dreidimensionale Raumnetzstruktur 6 trägt, wo Kontakt mit dem Knochenmaterial der benachbarten Wirbelkörper entsteht. Der dazwischenliegende Bereich liegt im Bereich der Bandscheibe und wird gebildet durch die elastische Einlage 4, welche an dieser Stelle die Aufgaben der natürlichen Bandscheibe wahrnimmt, d. h. als Puffer wirkt.

Operativ geht der Operateur so vor, daß er in die lädierte Bandscheibe mindestens zwei Bohrungen einbringt mit einem Durchmesser, der größer ist als die Bandscheibendicke, so daß also auch die benachbarten Wirbelkörper angefräst werden mit einer Tiefe, die der Höhe H (Fig. 3) der Oberfläche entspricht, welche mit der dreidimensionalen, offenmaschigen Raumnetzstruktur 6 belegt ist.

## Patentansprüche

1. Bandscheibenteilersatzimplantat (1), bestehend aus einer im wesentlichen zylindrischen, zweigeteilten Hülse und aus einer Einlage (4) aus elastischem Material, welche die beiden Hülsenteile (2, 3) voneinander trennt und zwischen diesen einen elastischen Puffer bildet, **dadurch gekennzeichnet, daß** die Teile (2, 3) der Hülse einseitig aneinander angelenkt sind und daß das distale Ende (5) ballig ausgebildet ist.

2. Bandscheibenteilersatzimplantat nach Anspruch 1, bei dem die beiden Hülsenteile (2, 3) wenigstens im Bereich des cranialen und caudalen Kontaktbereiches mit Knochenmaterial mit einer offenmaschigen, dreidimensionalen Raumnetzstruktur (6) versehen ist.

3. Bandscheibenteilersatzimplantat nach Anspruch 1 oder 2, bei dem die Einlage (4) einen Mittelwulst (7) aufweist, der in entsprechend ausgebildete Mulden (8) in den beiden Hülsenhälften (2, 3) formschlüssig eingreift.

4. Bandscheibenteilersatzimplantat nach einem der Ansprüche 1 bis 3, bei dem die Einlage (4) aus körperverträglichem Silikon mit einer Shore-Härte im Bereich vom 35-70 besteht.

5. Bandscheibenteilersatzimplantat nach einem der Ansprüche 1 bis 4, bei dem am proximalen Ende eine Aufnahme für ein Setzinstrument angeordnet ist.

## Claims

1. Partial intervertebral disk replacement implant (1) consisting of an essentially cylindrical, two-part sleeve and of an insert (4) made from resilient material, which separates the two sleeve parts (2, 3) from one another and forms between the latter a resilient buffer, **characterised in that** the parts (2, 3) of the sleeve are hinged to one another on one side and **in that** the distal end (5) is designed to be spherical.

2. Partial intervertebral disk replacement implant according to claim 1, in which the two sleeve parts (2, 3) are provided with an open-mesh, three-dimensional spatial network structure (6) at least in the region of the cranial and caudal contact region with bone material.

3. Partial intervertebral disk replacement implant according to claim 1 or 2, in which the insert (4) has a central bead (7) which engages positively in correspondingly designed depressions (8) in the two sleeve halves (2, 3).

4. Partial intervertebral disk replacement implant according to one of claims 1 to 3, in which the insert (4) consists of biocompatible silicon having a Shore hardness in the range from 35-70.

5. Partial intervertebral disk replacement implant according to one of claims 1 to 4, in which a recess for a set instrument is arranged on the proximal end.

## Revendications

1. Implant pour disque intervertébral (1), formé par un manchon sensiblement cylindrique, séparé en deux parties, et un corps interne (4) en matériau élastique, qui sépare l'une de l'autre les deux parties du manchon (2, 3) et qui forme un tampon élastique entre celles-ci, **caractérisé en ce que** les parties (2, 3) du manchon sont articulées l'une contre l'autre sur un côté et **en ce que** l'extrémité distale (5) est conçue avec une forme convexe.

2. Implant pour disque intervertébral selon la revendication 1, dans lequel les deux parties du manchon (2, 3), au moins dans la région de la zone de contact antérieure et caudale avec la matière osseuse, sont munies d'une structure réticulaire (6) tridimensionnelle à mailles ouvertes.

3. Implant pour disque intervertébral selon la revendication 1 ou 2, dans lequel le corps interne (4) comporte un bourrelet central (7) qui s'engage par conjugaison de forme dans des cavités de forme correspondante, ménagées dans les deux moitiés du manchon (2, 3).

4. Implant pour disque intervertébral selon l'une quelconque des revendications 1 à 3, dans lequel le corps interne (4) est réalisé en silicone tolérée par le corps humain, avec une dureté Shore dans le domaine de 35-70.

5. Implant pour disque intervertébral selon l'une quelconque des revendications 1 à 4, dans lequel un logement pour un instrument de pose est agencé au niveau de l'extrémité proximale.
